# EUROPÄISCHE PATENTSCHRIFT

(11) **EP 1 771 406 B1**
(45) Veröffentlichungstag und Bekanntmachung des Hinweises auf die Patenterteilung: **19.04.2017**
(21) Anmeldenummer: 05770772.1
(22) Anmeldetag: 15.07.2005
(51) Int. Cl.: C07C 51/43, C07C 57/04

(54) **EIN VERFAHREN ZUR REINIGUNG VON (METH) ACRYLSÄURE**
METHOD FOR THE PURIFICATION OF (METH)ACRYLIC ACID
PROCEDE DE PRODUCTION D'ACIDE (METH)ACRYLIQUE

(30) Priorität: 15.07.2004 DE 102004034316
(43) Veröffentlichungstag der Anmeldung: 11.04.2007
(73) Patentinhaber: Evonik Degussa GmbH, 45128 Essen (DE)
(72) Erfinder: NORDHOFF, Stefan, 45657 Recklinghausen (DE); BALDUF, Torsten, Houston, TX 77062-2133 (US); MOSLER, Jürgen, 44577 Castrop-Rauxel (DE); UHLICH, Wilfried, 45768 Marl (DE); THONG YU-CHIANG, Dennis, 45772 Marl (DE); KOBUS, Axel, 32225 Langen (DE); SELBACH, Arndt, 67246 Dirmstein (DE); KOHN, Jürgen, No. 01-27 589648 (SG)
(74) Vertreter: Lang, Arne
(86) Internationale Anmeldenummer: PCT/EP2005/007719
(87) Internationale Veröffentlichungsnummer: WO 2006/008083

(56) Entgegenhaltungen:
- EP-A- 0 616 998
- WO-A-00/45928
- WO-A-03/014172

## Beschreibung

Die vorliegende Erfindung betrifft ein Verfahren zur Herstellung von (Meth)Acrylsäure, die durch dieses Verfahren erhältliche (Meth)Acrylsäure, eine Vorrichtung zur Herstellung von (Meth)Acrylsäure, die Verwendung der Vorrichtung oder einer Aufreinigungsvorrichtung zur Herstellung von (Meth)Acrylsäure, (Meth)Acrylsäure, wasserabsorbierende Polymere, die Verwendung wasserabsorbierender Polymere sowie Fasern, Folien, Schäume und Verbunde.

"(Meth)Acrylsäure" wird in diesem Text für die Verbindungen mit den Nomenklaturnamen "Methacrylsäure" und "Acrylsäure" verwendet. Von beiden Verbindungen ist die Acrylsäure sowohl im Zusammenhang mit dem erfindungsgemäßen Verfahren als auch im Zusammenhang mit den erfindungsgemäßen chemischen Produkten bevorzugt.

Chemische Ausgangsprodukte und Zwischenprodukte, insbesondere Ausgangsprodukte für die Polymerherstellung, werden heutzutage in sehr großen Mengen hergestellt. Um den steigenden Qualitätsanforderungen gerecht zu werden, dürfen die Produkte praktisch keine Verunreinigungen aufweisen. Dieses gilt in besonderer Weise für Acrylsäure, wenn diese zur Herstellung wasserabsorbierender Polymere, die beispielsweise in Hygieneartikeln eingesetzt werden, Verwendung findet. Verunreinigungen in der Acrylsäure beeinflussen nachhaltig deren Polymerisation. So nimmt mit zunehmendem Anteil an Verunreinigungen in der Acrylsäure nicht nur der Anteil an Restmonomeren in den Acrylsäurepolymeren zu, auch das Absorptionsverhalten der auf Acrylsäure basierenden wasserabsorbierenden Polymere wird durch Verunreinigungen in der eingesetzten Acrylsäure nachteilig beeinflusst.

Acrylsäure wird üblicherweise durch katalytische Gasphasenoxidation von Propylen mit einem sauerstoffhaltigen Gas erhalten. Dabei wird in einem zweistufigen Prozess das Propylen zunächst auf katalytischem Weg zu Acrolein oxidiert, welches anschließend in einer zweiten Verfahrensstufe ebenfalls unter Einsatz von Katalysatoren zur Acrylsäure umgesetzt wird. Die so erhaltene Acrylsäure wird durch Absorption mit Wasser in Form einer wässrigen Lösung aus dem gasförmigen Reaktionsgemisch entfernt. Das Wasser in der so erhaltenen wässrigen Acrylsäure wird anschließend unter Bildung einer Roh-Acrylsäure durch Destillation, vorzugsweise mittels eines Schleppmittels, abgetrennt, wobei ein acrylsäurehaltiges Sumpfprodukt (= Roh-Acrylsäure) erhalten wird.

Methacrylsäure kann analog zur Acrylsäure ebenfalls durch katalytische Gasphasenoxidation hergestellt werden, wobei in diesem Fall C₄-Ausgangsverbindungen wie beispielsweise Isobuten, Isobutan, tert.-Butanol oder Methacrolein eingesetzt werden. Die Aufreinigung der durch die katalytische Gasphasenoxidation von C₄-Ausgangsverbindungen erhaltenen Methacrylsäure umfasst, wie bei der Aufreinigung der Acrylsäure, die Absorption der Methacrylsäure in einem geeigneten Lösungsmittel sowie das anschließende Abtrennen des Lösungsmittels, wobei in diesem Fall eine Roh-Methacrylsäure erhalten wird.

Da sowohl bei der katalytischen Gasphasenoxidation der C₃- oder C₄-Ausgangsverbindungen neben der Acrylsäure bzw. der Methacrylsäure auch andere Oxidationsprodukte, wie beispielsweise Maleinsäureanhydrid, entstehen, die im Falle der Herstellung wasserabsorbierender Polymere auf der Basis von Polyacrylaten die Polymerisation inhibieren und sich nachteilig auf die Absorptionseigenschaften der wasserabsorbierenden Polymere auswirken können, ist eine weitere Aufreinigung der Roh-(Meth)Acrylsäure notwendig. Die Aufreinigung der Roh-(Meth)Acrylsäure erfolgt dabei mittels der aus dem Stand der Technik bekannten Aufreinigungsverfahren, beispielsweise mittels Destillation oder Kristallisation, wobei im Falle der Kristallisation technisch zwischen den beiden Verfahren der Suspensionskristallisation und der Schichtkristallisation unterschieden wird (Wintermantel et al., Chem. Ing. Tech. 1991, 63, 881-891; Steiner et al., Chem. Ing. Tech. 1985, 57, 91-102).

Die Kristallisation hat gegenüber der Destillation den Vorteil, dass Verunreinigungen, die sich destillativ nicht entfernen lassen, häufig mittels Kristallisation abgetrennt werden können. Außerdem kann die Kristallisation im Vergleich zur Destillation bei deutlich niedrigeren Temperaturen durchgeführt werden, so dass das Ausmaß der Bildung von (Meth)Acrylsäure-Dimeren oder (Meth)Acrylsäure-Oligomeren während des Aufreinigungsverfahrens reduziert werden kann. Die Kristallisation hat jedoch den Nachteil, dass auch gegebenenfalls in der Schmelze vorhandene Verunreinigungen, insbesondere Fumarsäure, Maleinsäure und Maleinsäureanhydrid, beim Kristallisieren ausfallen. Da während der Kristallisation die Mutterlauge oder die Schmelze an der (Meth)Acrylsäure verarmt, reichern sich Verunreinigungen in der Mutterlauge stetig an. Sobald die Löslichkeitsgrenze erreicht ist, fallen diese Verbindungen aus der Mutterlauge in kristalliner Form aus. Im Falle einer statischen Schichtkristallisation setzen sich diese unerwünschten Kristalle am Boden oder den Wänden eines Kristallisators ab. Da die Kristalle an den Oberflächen haften, bleiben diese beim Ablassen der nicht-kristallisierten Schmelze im Kristallisator zurück. Wird dann die abgeschiedene Kristallschicht abgeschmolzen, wobei üblicherweise zwei oder mehrere Fraktionen gebildet werden, so werden die am Boden und den Wänden des Kristallisators noch vorhandenen, ausgefallenen kristallinen Verunreinigungen von der erwännten Schmelze wieder aufgenommen. Die Folge davon ist, dass diese Verunreinigungen sich nie vollständig entfernen lassen und sich im Kristallisator aufkonzentrieren. Bei einer großtechnischen Reinigungsanlage können so im Laufe eines Tages beachtliche Mengen an Verunreinigungen, wie beispielsweise Maleinsäure, anfallen, welche die Leitungen und Ventile der Aufreinigungsvorrichtung für (Meth)Acrylsäure verstopfen kann. Auch können sich Verunreinigungen als fester Belag an den Kristallisatorwänden oder am Boden niederschlagen. Diese Ablagerungen führen nicht nur zu Verstopfungen, sondern bewirken auch eine Reduktion der Kapazität einer Anlage zur Aufreinigung von (Meth)Acrylsäure, da ein beachtlicher Teil des Volumens beispielsweise der in der Anlage befindlichen Tanks von den ausgefallenenen Kristallen beansprucht wird.

Es hat sich deshalb als eine Notwendigkeit erwiesen, die Ausfällungen abzutrennen.

Dabei schlägt WO-A-00/45928 vor, das Ausfallen von Verunreinigungen im Reinigungsverfahren für Acrylsäure durch den Zusatz eines Lösungsmittels oder eines Lösungsmittelgemisches zu verhindern, wobei das Lösungsmittel oder das Lösungsmittelgemisch in einer solchen Menge zugesetzt wird, dass die Verunreinigungen in Lösung gehalten werden. Vorzugsweise wird als Lösungsmittel Wasser eingesetzt. Der Nachteil dieses Verfahrens besteht jedoch darin, dass Maleinsäure und Maleinsäureanhydrid durch dieses Verfahren nicht aus der Mutterlauge abgetrennt werden, sondern sich stetig in der Mutterlauge anreichern. Die Verdünnung der Mutterlauge mit dem Lösungsmittel oder dem Lösungsmittelgemisch hat zudem zur Folge, dass in nachgeschalteten Kristallisatoren eine Kristallisation der Acrylsäure durch den Eintrag eines Lösungsmittels erschwert wird.

Der vorliegenden Erfindung lag demnach die Aufgabe zugrunde, ein Verfahren anzugeben, mit welchem die im Stand der Technik beschriebenen Nachteile überwunden werden können.

Der vorliegenden Erfindung lag insbesondere die Aufgabe zugrunde, ein Verfahren zur Herstellung von (Meth)Acrylsäure anzugeben, welches ein Aufreinigungsverfahren für (Meth)Acrylsäure umfasst, bei dem in einfacher Weise Verunreinigungen, insbesondere Fumarsäure, Maleinsäure oder Maleinsäureanhydrid, abgetrennt werden können, ohne dass eine nachfolgende Kristallisation der (Meth)Acrylsäure erschwert wird.

Auch war es eine Aufgabe der vorliegenden Erfindung, eine (Meth)Acrylsäure anzugeben, die gegenüber der im Stand der Technik beschriebenen (Meth)Acrylsäure durch einen besonders geringen Gehalt an Verunreinigungen, wie etwa Fumarsäure, Maleinsäure oder Maleinsäureanhydrid, gekennzeichnet ist.

Weiterhin lag der vorliegenden Erfindung die Aufgabe zugrunde, wasserabsorbierende Polymere anzugeben, die durch einen besonders geringen Gehalt an Verunreinigungen, wie etwa Fumarsäure, Maleinsäure oder Maleinsäureanhydrid, und somit durch eine besonders vorteilhafte Hautverträglichkeit gekennzeichnet sind. Gelöst wurden diese Aufgaben durch ein Verfahren zur Herstellung von (Meth)Acrylsäure, wobei zunächst eine Roh-(Meth)Acrylsäure hergestellt und diese Roh-(Meth)Acrylsäure anschließend vorzugsweise kontinuierlich aufgereinigt wird, wobei die vorzugsweise kontinuierliche Aufreinigung der Roh-(Meth)Acrylsäure folgende Verfahrensschritte beinhaltet:
a) in einer Zusammensetzung beinhaltend (Meth)Acrylsäure und Verunreinigungen werden die Verunreinigungen in kristalliner Form aus der Zusammensetzung ausgefällt;
b) die aus der Zusammensetzung ausgefallenen kristallinen Verunreinigungen werden abgetrennt, wobei das Ausfällen des Maleinsäureanhydrids im Schritt a) durch kontrollierte Hydrolyse des Maleinsäureanhydrids über eine Zeitraum von 1 bis 250 Stunden erfolgt.

In einer bevorzugten Ausführungsform des erfindungsgemäßen Verfahrens handelt es sich bei der Verunreinigung um Maleinsäureanhydrid, Maleinsäure, Fumarsäure oder um Mischungen dieser Verbindungen. In einer anderen bevorzugten Ausführungsform des erfindungsgemäßen Verfahrens handelt es sich bei den Verunreinigungen um Polymerisationsinhibitoren wie etwa Phenothiazin, Hydrochinonmonomethylether oder Hydrochinon, die bei der Herstellung von (Meth)Acrylsäure zugesetzt werden, um eine Bildung von (Meth)AcrylsäurePolymeren während des Synthese- und des darauf folgenden Aufreinigungsprozesses zu verhindern.

Wenn es sich bei der Verunreinigung um Maleinsäureanhydrid handelt, so erfolgt die Abtrennung des Maleinsäureanhydrids aus der Zusammensetzung vorzugsweise dadurch, dass im Verfahrensschritt a) Maleinsäureanhydrid zu mindestens 50 %, vorzugsweise zu mindestens 75 %, besonders bevorzugt zu mindestens 96 % und darüber hinaus bevorzugt zu mindestens 99,99 % unter Bildung von Maleinsäure hydrolysiert wird, wobei Maleinsäure und/oder Fumarsäure in kristalliner Form aus der Zusammensetzung ausfallen.

Überraschend, aber nicht minder vorteilhaft, wurde festgestellt, dass aus Zusammensetzungen beinhaltend (Meth)Acrylsäure und Maleinsäureanhydrid das Maleinsäureanhydrid durch Hydrolyse und der daraus resultierenden Bildung von Maleinsäure (welche unter Umständen zumindest teilweise in einer langsamen Isomerisierung in Fumarsäure umgewandelt wird), in einfacher Weise abgetrennt werden kann, da Maleinsäure (und auch Fumarsäure) im Vergleich zum Maleinsäureanhydrid in (Meth)Acrylsäure schwerer löslich sind. Dabei wurde durch intensive Untersuchungen an Zusammensetzungen mit einem unterschiedlichen Mengenverhältnis von (Meth)Acrylsäure, Maleinsäureanhydrid und Wasser auch festgestellt, dass mitunter Stunden oder auch Tage vergehen können, bis eine Ausfällung von Maleinsäureanhydrid oder von durch die Hydrolyse des Maleinsäureanhydrids entstehender Maleinsäure und/oder Fumarsäure zu beobachten ist.

Um die für eine ausreichende Abtrennung des Maleinsäureanhydrids möglichst vollständige Hydrolyse des Anhydrids zu gewährleisten, ist es erfindungsgemäß bevorzugt, dass die Hydrolyse des Maleinsäureanhydrids im Verfahrensschritt a) vorzugsweise über einen Zeitraum in einem Bereich von 1 bis 250 Stunden, besonders bevorzugt über einen Zeitraum von 2 bis 100 Stunden und darüber hinaus noch mehr bevorzugt über einen Zeitraum von 5 bis 50 Stunden und vorzugsweise bei einer Temperatur in einem Bereich von -70 bis 130°C, besonders bevorzugt in einem Bereich von -30 bis 100°C und darüber hinaus bevorzugt in einem Bereich von -10 bis 20°C erfolgt. Die Dauer des Hydrolysevorganges hängt dabei von unterschiedlichen Faktoren wie etwa der Temperatur der Zusammensetzung, dem relativen Mengenverhältnissen der einzelnen Komponenten (Wasser, (Meth)Acrylsäure, andere Verunreinigungen) in der Zusammensetzung sowie von der Anwesenheit von die Hydrolyse des Maleinsäureanhydrids beschleunigenden Katalysatoren ab. Der Fachmann wird die notwendige Dauer des Hydrolysevorganges durch entsprechende Routineversuche bestimmen, bei denen stichproben-artig das Fortschreiten der Hydrolyse in der Zusammensetzung, beispielsweise durch die Bestimmung des Wassergehaltes (dieser nimmt mit fortschreitender Hydrolyse ab) kontrolliert wird.

Vorzugsweise erfolgt im Falle von Maleinsäureanhydrid als abzutrennender Verunreinigung die kontrollierte Hydrolyse der Verunreinigung, in dem sichergestellt wird, dass, abhängig vom pH-Wert der Zusammensetzung und der Konzentration des in der Zusammensetzung enthaltenen Maleinsäureanhydrids, eine für das geforderte Ausmaß der Hydrolyse ausreichende Menge an Wasser in der Zusammensetzung enthalten ist. Dabei beinhaltet die Zusammensetzung das Wasser vorzugsweise in einer Menge in einem Bereich von 0,01 bis 50 Gew.-%, besonders bevorzugt in einer Menge in einem Bereich von 0,1 bis 20 Gew.-%, darüber hinaus bevorzugt in einem Bereich von 1 bis 10 Gew.-%, und darüber hinaus noch mehr bevorzugt in einem Bereich von 2 bis 5 Gew.%, jeweils bezogen auf das Gesamtgewicht der Zusammensetzung. Vorzugsweise ist in der Zusammensetzung jedoch die Menge an Wasser dadurch begrenzt, dass höchstens 10 Gew.-%, besonders bevorzugt höchstens 5 Gew.-%, darüber hinaus bevorzugt höchstens 1 Gew.-% und darüber hinaus noch mehr bevorzugt höchstens 0,1 Gew.-% der Maleinsäure, jeweils bezogen auf das Gewicht der Maleinsäure in der Zusammensetzung, in im Wasser gelöster Form vorliegen, wobei diese jeweilige Höchstmenge an Wasser von der Temperatur der Zusammensetzung abhängt.

In einer besonders bevorzugten Ausführungsform des erfindungsgemäßen Verfahrens beinhaltet die Zusammensetzung Wasser in einer solchen Menge, dass das in der Zusammensetzung enthaltene Maleinsäureanhydrid zu mindestens 50 %, vorzugsweise zu mindestens 75 %, besonders bevorzugt zu mindestens 96 % und darüber hinaus bevorzugt zu mindestens 99,99 % unter Bildung von Maleinsäure und/oder Fumarsäure hydrolysiert wird. Dabei wird der Fachmann die benötigte Wassermenge anhand einfacher Kontrollversuche, bei denen die Menge an Maleinsäureanhydrid in der Zusammensetzung und somit das Fortschreiten der Hydrolyse bestimmt wird, ermitteln.

Sofern in dem bevorzugten erfindungsgemäßen Verfahren, bei dem Maleinsäureanhydrid als Verunreinigung abgetrennt werden soll, im Verfahrensschritt a) eine Zusammensetzung eingesetzt wird, welche die erforderliche Wassermenge bereits aufweist, so wird sich im Gleichgewichtszustand der erforderliche Hydrolysegrad einstellen und die Maleinsäure und/oder Fumarsäure aus der Zusammensetzung auskristallisieren. Sollte die in der Zusammensetzung enthaltene Wassermenge jedoch nicht ausreichen, um auch nach entsprechenden Wartezeiten den benötigten Hydrolysegrad zu ermöglichen, so ist es erfindungsgemäß bevorzugt, der Zusammensetzung eine solche Menge Wasser zuzusetzen, dass im Gleichgewichtszustand der gewünschte Hydrolysegrad erreicht und somit die Maleinsäure und/oder Fumarsäure zur Ausfällung gebracht wird. Neben dem Zusatz von Wasser kann jedoch der erforderliche Hydrolysegrad auch durch die Zugabe von die Hydrolyse von Maleinsäureanhydrid beschleunigenden Katalysatoren, wie etwa anorganischen oder organischen Säuren, oder aber durch Variation anderer Verfahrensparameter wie etwa einer Erhöhung der Temperatur der Zusammensetzung, eingestellt werden. Im Falle eines Einsatzes von Katalysatoren werden diese vorzugsweise in einer Menge in einem Bereich von 1 bis 10.000 ppm, besonders bevorzugt in einem Bereich von 10 bis 5.000 ppm und darüber hinaus bevorzugt in einem Bereich von 100 bis 1.000 ppm, jeweils bezogen auf die Gesamtmenge an Maleinsäureanhydrid in der Zusammensetzung, eingesetzt.

Erfolgt die Aufreinigung der Zusammensetzung beinhaltend (Meth)Acrylsäure und Maleinsäureanhydrid als Verunreinigung diskontinuierlich, in dem beispielsweise die Zusammensetzung in einem Behältnis gelagert wird, so wird die Zusammensetzung vorzugsweise für die vorstehend genannten Zeiträume in diesem Behältnis gelagert und anschließend einer entsprechenden Vorrichtung zur Abtrennung der ausgefallenen Maleinsäure und/oder Fumarsäure, beispielsweise einem Filter, zugeführt. Erfolgt die Aufreinigung der Zusammensetzung beinhaltend (Meth)Acrylsäure und Maleinsäureanhydrid kontinuierlich, indem beispielsweise die Zusammensetzung mittels geeigneter Kristallisatoren in eine Kristallsuspension überführt und diese Kristallsuspension anschließend in Waschkolonnen in (Meth)Acrylsäurekristalle und eine Mutterlauge getrennt werden, so kann eine ausreichende Hydrolyse des in der Mutterlauge verbleibenden Maleinsäureanhydrids beispielsweise dadurch sichergestellt werden, dass die Mutterlauge durch teilweise Rückführung in den Kristaller für eine genügend lange Zeit im Kreislauf geführt wird.

Erfindungsgemäß werden demnach Verunreinigungen, wie etwa Maleinsäure bzw. Maleinsäureanhydrid, nicht, wie in der WO-A-00/45928 beschrieben, in Lösung gehalten, sondern aus der Zusammensetzung ausgefällt, wobei im Falle von Maleinsäureanhydrid als abzutrennende Verunreinigung dieses Ausfällen durch die kontrollierte Hydrolyse des Maleinsäurenhydrids erfolgt. Da bei der Abtrennung von Maleinsäureanhydrid als Verunreinigung gegenüber dem in der WO-A-00/45928 beschriebenen Verfahren hierzu allenfalls geringe Zusätze an Wasser erforderlich sind, ermöglicht das erfindungsgemäße Verfahren insbesondere die gezielte Abtrennung von Maleinsäureanhydrid aus einer (Meth)Acrylsäure enthaltenden Zusammensetzung, ohne diese Zusammensetzung entscheidend mit Wasser zu verdünnen und so eine in nachfolgenden Reinigungsschritten durchgeführt weitere Kristallisation von (Meth)Acrylsäure aus dieser Zusammensetzung zu erschweren.

Das Abtrennen der Maleinsäure und/oder der Fumarsäure im Verfahrensschritt b) kann durch jedes Verfahren, welches eine Trennung von festen und flüssigen Stoffen erlaubt, durchgeführt werden. Vorzugsweise erfolgt das Abtrennen der kristallisierten Maleinsäure und/oder der kristallinen Fumarsäure durch Filtrieren, Sedimentieren oder Zentrifugieren.

In einer bevorzugten Ausführungsform des erfindungsgemäßen Verfahrens handelt es sich bei der Zusammensetzung beinhaltend (Meth)Acrylsäure und die Verunreinigungen gemäß Verfahrensschritt a) um das Sumpfprodukt S2, welches erhalten wird, indem die als Quenchphase erhaltene wässrige (Meth)Acrylsäurelösung, vorzugsweise Acrylsäurelösung, in einem ersten Destillationsschritt in Gegenwart eines Schleppmittels, vorzugsweise in Gegenwart von Toluol, destilliert wird, um der Quenchphase das Wasser zu entziehen. Das in diesem ersten Destillationsschritt erhaltene, (meth)acrylsäurehaltige, im Vergleich zur Quenchphase wasserarme Sumpfprodukt S1 wird in einem zweiten Destillationsschritt destilliert, um leichtsiedende Komponenten, wie etwa Essigsäure, abzutrennen. Bei diesem zweiten Destillationsschritt wird das (meth)acrylsäurehaltige Sumpfprodukt S2 (= verunreinigter Roh-(Meth)Acrylsäurestrom) erhalten. Die Abtrennung von Maleinsäureanhydrid, Maleinsäure oder Fumarsäure aus dem Sumpfprodukt S2 kann dabei erfolgen, noch bevor in dem Sumpfprodukt S2 vorhandene (Meth)Acrylsäure beispielsweise mittels Kristallisation abgetrennt wird. Denkbar ist auch, zunächst noch im Sumpfprodukt S2 vorhandene (Meth)Acrylsäure durch Kristallisation abzutrennen und aus der so zurückbehaltenen Mutterlauge Maleinsäure, Maleinsäureanhydrid oder Fumarsäure abzutrennen. Die Mutterlauge kann dann nachfolgenden Reinigungsstufen zum Zwecke einer weiteren Abtrennung von noch vorhandener (Meth)Acrylsäure zugeführt werden.

In einer anderen bevorzugten Ausführungsform des erfindungsgemäßen Verfahrens handelt es sich bei der Zusammensetzung beinhaltend (Meth)Acrylsäure und die Verunreinigungen gemäß Verfahrensschritt a)
I. um die (Meth)Acrylsäurekristalle, vorzugsweise die mindestens teilweise geschmolzenen (Meth)Acrylsäurekristalle, oder die Mutterlauge, die im Schritt B oder im Schritt D oder
II. um die Kristallsuspension, die im Schritt A oder Schritt C eines Verfahrens erhalten werden, welches die folgenden Verfahrenschritte beinhaltet:
   A) Kristallisation von (Meth)Acrylsäure aus einem verunreinigten Roh-(Meth)Acrylsäurestrom, vorzugsweise aus dem Sumpfprodukt S2, aus einem Verfahren zur Herstellung von (Meth)Acrylsäure, vorzugsweise unter Bildung einer Kristallsuspension mittels eines ersten Suspensionserzeugers;
   B) Abtrennen der (Meth)Acrylsäurekristalle aus der Kristallsuspension, vorzugsweise mittels einer ersten Abtrennvorrichtung, vorzugsweise einer ersten Waschkolonne, wobei eine Mutterlauge zurückbehalten wird; sowie gegebenenfalls
   C) erneute Kristallisation von (Meth)Acrylsäure aus der im Schritt B) erhaltenen ersten Mutterlauge, vorzugsweise unter Bildung einer zweiten Kristallsuspension mittels eines zweiten Suspensionserzeugers;
   D) Abtrennen der im Schritt C) erhaltenen (Meth)Acrylsäurekristalle aus der zweiten Kristallsuspension, vorzugsweise mittels einer weiteren Abtrennvorrichtung, vorzugsweise einer weiteren Waschkolonne, wobei eine zweite Mutterlauge zurückbehalten wird.

Erfindungsgemäß sind unter dem Begriff "Mutterlauge" in dieser Schrift alle Phasen zu verstehen, die nicht in kristalliner Form vorliegen. Im Falle einer Kristallsuspension wird demnach unter dem Begriff "Mutterlauge" diejenige flüssige Phase verstanden, die beim Abtrennen der Kristalle aus der Kristallsuspension zurückbehalten wird.

Der in dieser besonderen Ausführungsform des erfindungsgemäßen Verfahrens im Verfahrensschrift A) eingesetzte, verunreinigte Roh-(Meth)Acrylsäurestrom basiert vorzugsweise auf
(α1) (Meth)Acrylsäure in einer Menge in einem Bereich von 50 bis 99,9 Gew.-%, besonders bevorzugt in einem Bereich von 60 bis 99,6 Gew.-% und darüber hinaus bevorzugt in einer Menge in einem Bereich von 90 bis 99,5 Gew.-%,
(α2) Wasser in einer Menge in einem Bereich von 0,01 bis 50 Gew.-%, besonders bevorzugt in einem Bereich von 0,03 bis 10 Gew.-% und darüber hinaus bevorzugt in einer Menge in einem Bereich von 0,05 bis 1 Gew.-%,
(α3) Maleinsäureanhydrid in einer Gesamtmenge in einem Bereich von 0,01 bis 5 Gew.-%, besonders bevorzugt in einem Bereich von 0,05 bis 1 Gew.-% und darüber hinaus bevorzugt in einer Menge in einem Bereich von 0,1 bis 0,5 Gew.-%, sowie
(α4) weiteren Verunreinigungen in einer Menge in einem Bereich von 0,01 bis 5 Gew.-%, besonders bevorzugt in einem Bereich von 0,03 bis 1 Gew.-% und darüber hinaus bevorzugt in einer Menge in einem Bereich von 0,05 bis 0,5 Gew.-%, wobei die Gesamtmenge der Komponenten (α1) bis (α4) 100 Gew.-% beträgt.

Zu den weiteren Verunreinigungen (α4) gehören dabei im Falle der Herstellung von Acrylsäure Acrolein, Essigsäure, Propionsäure, Aldehyde wie beispielsweise Formaldehyd, Furfural und Benzaldehyd und Protoanemonin, sowie Polymerisationsinhibitoren wie etwa HQME (Hydrochinonmonomethylether), HQ (Hydrochinon) und Phenothiazin, die bei der weiteren, destillativen Aufreinigung des bei der katalytischen Gasphasenoxidation von Propylen erhaltenen Reaktionsproduktes zugesetzt werden, um die Polymerisation und die daraus resultierende Bildung von Acrylsäure-Dimeren oder Acrylsäure-Oligomeren zu verhindern. Im Falle der Herstellung von Methacrylsäure handelt es sich bei den weiteren Verunreinigungen (α4) beispielsweise um Hydroxy-iso-buttersäure, iso-Buttersäure, Methacrylamid, Methylmethacrylat, Methacrolein, Essigsäure, Acrylsäure, Propionsäure, HQME und HQ.

Im Falle eines zweistufigen Aufreinigungsverfahrens (Verfahrensschritt A bis D) ist es gemäß einer besonderen Ausführungsform dieses Verfahrens bevorzugt, dass im Verfahrensschritt A bereits eine Roh-(Meth)Acrylsäure eingesetzt wird, die zuvor in den Kopf der im Verfahrensschritt D eingesetzten Abtrennvorrichtung geleitet wurde, um auf diese Weise die in der zweiten Verfahrensstufe abgeschabten Produktkristalle als Kristallsuspension in den Suspensionserzeuger der Verfahrensstufe A zu führen. Diese Variante hat den energetischen Vorteil, auf ein Aufschmelzen in der zweiten Stufe verzichten zu können und die nun in der ersten Stufe vorhandenen Kristalle nicht noch einmal ausfrieren zu müssen.

In einer bevorzugten Ausführungsform des erfindungsgemäßen Verfahrens, bei dem eine der Zusammensetzungen I oder II im Verfahrensschritt a) eingesetzt wird, wird die Kristallisation als mehrstufige, vorzugsweise zweistufige Kristallisation durchgeführt (Verfahrensschritte A bis D). Zweckmäßigerweise werden mehrstufige Verfahren hierbei nach dem Gegenstromprinzip betrieben, bei dem nach der Kristallisation in jeder Stufe das Kristallisat von der Mutterlauge abgetrennt und die Mutterlauge in diejenige Stufe mit geringerer Temperatur zugeführt wird.

Das für die Kristallisation im Schritt I verwendete Kristallisationsverfahren und das für die Abtrennung im Schritt II verwendete Abtrennverfahren entsprechen vorzugsweise denjenigen Kristallisations- und Abtrennverfahren, die in der WO 99/14181 A1 beschrieben sind, auf deren Inhalt hiermit als Teil der Offenbarung Bezug genommen wird.

Insbesondere im Falle der Abtrennung von Maleinsäureanhydrid als Verunreinigung durch die eingangs beschriebene kontrollierte Hydrolyse des Maleinsäureanhydrids unter Bildung von Maleinsäure und/oder Fumarsäure kommen vorzugsweise Suspensionskristallisationsverfahren zum Einsatz, und zwar insbesondere diejenigen Suspensionskristallisationsverfahren, die es gestatten, das erfindungsgemäße Verfahren kontinuierlich zu gestalten. Die Suspensionskristallisation kann vorteilhafterweise in einem Rührkesselkristallisator, einem Kratzkristallisator, einem Kühlscheibenkristallisator, einer Kristallisierschnecke, einem Trommelkristallisator, einem Rohrbündelkristallisator oder dergleichen durchgeführt werden. Von besonderem Vorteil sind hier wiederum solche Kristallisatoren, die kontinuierlich betrieben werden können. Vorzugsweise sind dies die Kühlscheibenkristallisatoren oder Kratzkühler (siehe die Dissertation von Poschmann zur Suspensionskristallisation organischer Schmelzen und Nachbehandlung der Kristalle durch Schwitzen oder Waschen, Diss. Universität Bremen, Shaker Verlag, Aachen 1996). Ganz besonders bevorzugt wird zur Kristallisation ein Kratzkühler eingesetzt.

Vorteilhafterweise liegt die Temperatur der Schmelze während der Kristallisation zwischen -30 und +14°C, insbesondere zwischen -15 und +14°C. Der Feststoffgehalt im Kristallisator liegt vorteilhafterweise zwischen 0 und 85 g, bevorzugt zwischen 20 und 40 g Feststoff/100 g der Zusammensetzung.

Die Abtrennung der (Meth)Acrylsäurekristalle aus den in den Verfahrensschritten A und C erhaltenen Kristallsuspensionen erfolgt vorzugsweise mittels Waschkolonnen. In einer bevorzugten Ausführungsform dieses erfindungsgemäßen Verfahrens wird die Suspension in einer hydraulischen Waschkolonne im oberen Teil der Kolonne aufgegeben. Die Mutterlauge wird über einen Filter aus der Kolonne abgezogen, wodurch sich ein dichtgepacktes Kristallbett bildet. Das Kristallbett und die Mutterlauge strömen dabei in Richtung des Bodens der Waschkolonne. Am Boden der Kolonne befindet sich eine bewegte, vorzugsweise rotierende Kratzvorrichtung oder Kratzer, die aus dem dichtgepackten Kristallbett wieder eine Suspension erzeugt. Diese Suspension wird vorzugsweise durch einen Aufschmelzer, vorzugsweise einen Wärmetauscher, gepumpt und aufgeschmolzen. Ein Teil der Schmelze kann z. B. als Waschschmelze dienen; diese wird dann in die Kolonne zurückgepumpt und wäscht vorzugsweise das in entgegengesetzter Richtung wandernde Kristallbett aus, d. h. die kristallisierte (Meth)Acrylsäure wird im Gegenstrom von der zurückgeführten (Meth)Acrylsäure gewaschen. Die Waschschmelze bewirkt einerseits ein Waschen der Kristalle, andererseits kristallisiert die Schmelze auf den Kristallen zumindest teilweise aus. Die freiwerdende Kristallisationsenthalphie erwärmt das Kristallbett im Waschbereich der Kolonne. Dadurch wird ein dem Schwitzen der Kristalle analoger Reinigungseffekt erzielt. In diesem Zusammenhang sei auf die DE 102 42 746 A1 sowie die DE 101 49 353 A1 verwiesen, die hiermit als Referenz eingeführt werden und deren Offenbarungsgehalt bezüglich der Abtrennung von (Meth)Acrylsäurekristallen aus Kristallsuspensionen einen Teil des Offenbarungsgehaltes der vorliegenden Anmeldung darstellt.

In einer besonderen Ausführungsform dieses erfindungsgemäßen Verfahrens zur Herstellung von (Meth)Acrylsäure, bei dem lediglich ein einstufiges Aufreinigungsverfahren durchgeführt wird (Verfahrensschritte A und B), wird zumindest ein Teil der im Schritt B erhaltenen Mutterlauge dem Schritt A zurückgeführt. Auf diese Weise kann die Mutterlauge in einem Kreislauf geführt werden. Bei einer beabsichtigten Abtrennung von Maleinsäureanhydrid als abzutrennende Verunreinigung kann somit neben der Menge an Wasser in der Zusammensetzung auch über die Regulation der Menge an Mutterlauge, die dem Schritt A zurückgeführt wird, und somit über die Verweilzeit der Mutterlauge im vorzugsweise kontinuierlichen Aufreinigungsverfahren, das Ausmaß der Hydrolyse des Maleinsäureanhydrids beeinflusst werden.

Denkbar ist auch, zumindest einen Teil der im Verfahrensschritt B erhaltenen (Meth)Acrylsäurekristalle zur Impfung in kristalliner Form dem Verfahrensschritt A zuzuführen, wie dies ebenfalls in der DE 102 11 686 A1 beschrieben ist.

Wird dieses erfindungsgemäße Verfahren mit zweistufiger Aufreinigung betrieben (Verfahrensstufen A bis D), so ist es erfindungsgemäß bevorzugt, dass die im Verfahrensschritt B und/oder die im Verfahrensschritt D erhaltenen (Meth)Acrylsäurekristalle in kristalliner und/oder zumindest teilweise aufgeschmolzener Form oder aber die in den Verfahrensschritten C und D erhaltenen Mutterlaugen zumindest teilweise in eine der Verfahrensstufen A bis D zurückgeführt werden, wobei die genaue Art der Rückführung der kristallinen oder geschmolzenen (Meth)Acrylsäure oder der Mutterlaugen im Falle eines zweistufigen Aufreinigungsverfahrens in der DE 102 11 686 A1 beschrieben ist. Die dort im Falle eines zweistufigen Aufreinigungsverfahrens beschriebenen besonderen Ausführungsformen sind auch im Falle des erfindungsgemäßen Verfahrens zur Herstellung von (Meth)Acrylsäure, welches ein Aufreinigungsverfahren mit den Verfahrensstufen A bis D umfasst, besonders bevorzugt.

Wenn im erfindungsgemäßen Verfahren die im Verfahrensschritt B oder im Verfahrensschritt D abgetrennte Mutterlauge als Zusammensetzung beinhaltend (Meth)Acrylsäure und Verunreinigungen gemäß dem Verfahrensschritt a) eingesetzt wird, so wird die Mutterlauge zumindest teilweise direkt der entsprechenden Abtrennvorrichtung zur Abtrennung der Verunreinigungen gemäß dem Verfahrensschritt b), beispielsweise einem Filter, zugeführt.

Wenn im erfindungsgemäßen Verfahren die im Verfahrensschritt B oder im Verfahrensschritt D erhaltenen (Meth)Acrylsäurekristalle als Zusammensetzung beinhaltend (Meth)Acrylsäure und Verunreinigungen gemäß dem Verfahrensschritt a eingesetzt werden, so ist es erfindungsgemäß bevorzugt, dass die (Meth)Acrylsäurekristalle zumindest teilweise aufgeschmolzen und in aufgeschmolzener Form der Vorrichtung zur Abtrennung der Verunreinigungen zugeführt werden, um eine Abtrennung von (Meth)Acrylsäurekristallen in der Abtrennvorrichtung zur Abtrennung von Verunreinigungen und somit einen Ausbeuteverlust möglichst gering zu halten. Das Abtrennen der Verunreinigungen aus einer aufgeschmolzenen (Meth)Acrylsäure kann zweckmäßiger Weise dadurch realisiert werden, dass im Falle eines teilweisen Aufschmelzen von (Meth)Acrylsäurekristallen und Rückführung dieser aufgeschmolzenen (Meth)Acrylsäurekristalle in einen der Verfahrensschritte A bis D zur Erzielung einer guten Reinheit der (Meth)Acrylsäure (siehe vorstehende Ausführungen im Zusammenhang mit dem in der DE 102 11 686 A1 beschriebenen Verfahren) dieser Strom aufgeschmolzener (Meth)Acrylsäure entweder direkt oder mit Hilfe eines separaten Produktkreislaufes durch die Abtrennvorrichtung zur Abtrennung der Verunreinigungen geführt wird.

Wenn im erfindungsgemäßen Verfahren die im Verfahrensschritt A oder im Verfahrensschritt C erhaltene Kristallsuspension als Zusammensetzung beinhaltend (Meth)Acrylsäure und Verunreinigungen gemäß dem Verfahrensschritt a) eingesetzt wird, so ist es erfindungsgemäß bevorzugt, dass die in diesen Verfahrensschritten erhaltene Kristallsuspension zumindest teilweise aufgeschmolzen und in aufgeschmolzener Form durch die Abtrennvorrichtung zur Abtrennung der Verunreinigungen geführt wird. Anschließend kann die aufgeschmolzene Kristallsuspension wieder dem Kristallisator zugeführt werden.

Im Falle eines einstufigen Aufreinigungsverfahrens (Verfahrensschritte A und B) wird als Zusammensetzung beinhaltend (Meth)Acrylsäure und Verunreinigungen, vorzugsweise Maleinsäureanhydrid, gemäß dem Verfahrensschritt a) vorzugsweise eine Zusammensetzung ausgewählt aus:
(1) der Mutterlauge, die im Verfahrensschritt B erhalten wurde, sofern diese zumindest teilweise in den Verfahrensschritt A zurückgeführt wird;
(2) den zumindest teilweise aufgeschmolzenen (Meth)Acrylsäurekristallen, die im Verfahrensschritt B erhalten wurden;
(3) der zumindest teilweise aufgeschmolzenen Kristallsuspension, die im Schritt A erhalten wurde.

Neben einer Abtrennung von Verunreinigungen aus nur einer der Zusammensetzungen (1), (2) oder (3) kann selbstverständlich im Falle eines einstufigen Aufreinigungsverfahrens auch eine Abtrennung der Verunreinigungen an mehreren Stellen des Aufreinigungsverfahrens bzw. aus mehreren, unterschiedlichen Zusammensetzungen erfolgen. So ist denkbar, die Verunreinigungen sowohl aus der Mutterlauge (Zusammensetzung (1)) als auch aus den teilweise aufgeschmolzenen (Meth)Acrylsäurekristallen (Zusammensetzung (2)) abzutrennen. Auch eine Abtrennung aus allen Zusammensetzungen (1) bis (3) ist möglich. Am meisten bevorzugt ist eine Abtrennung aus der Mutterlauge, die im Verfahrensschritt B erhalten wurde, sofern diese zumindest teilweise in den Verfahrensschritt A zurückgeführt wird.

Im Falle eines zweistufigen Aufreinigungsverfahrens (Verfahrensschritte A bis D) wird als Zusammensetzung beinhaltend (Meth)Acrylsäure und Verunreinigungen gemäß dem Verfahrensschritt a vorzugsweise eine Zusammensetzung ausgewählt aus:
(4) der Mutterlauge, die im Verfahrensschritt B erhalten wurde und die dem Verfahrensschritt C zugeführt wird;
(5) der Mutterlauge, die im Verfahrensschritt B erhalten wurde und die zumindest teilweise in den Verfahrensschritt A zurückgeführt wird;
(6) der Mutterlauge, die im Verfahrensschritt D erhalten wurde, sofern diese Mutterlauge zumindest teilweise, vorzugsweise in den Verfahrensschritt C, zurückgeführt wird;
(7) den zumindest teilweise aufgeschmolzenen (Meth)Acrylsäurekristallen, die im Verfahrensschritt B erhalten wurden;
(8) den vorzugsweise zumindest teilweise aufgeschmolzenen (Meth)Acrylsäurekristallen, die im Verfahrensschritt D erhalten wurden und die zumindest teilweise in den Verfahrensschritt A zurückgeführt werden;
(9) der Kristallsuspension, vorzugsweise der zumindest teilweise aufgeschmolzenen Kristallsuspension, die im Schritt A erhalten wurde;
(10) der Kristallsuspension, vorzugsweise der zumindest teilweise aufgeschmolzenen Kristallsuspension, die im Schritt C erhalten wurde;
(11) der Roh-(Meth)Acrylsäure, die im Verfahrensschritt A eingesetzt wird und zuvor in den Kopf der zweiten Waschkolonne geleitet wurde.

Auch im Hinblick auf dieses zweistufige Aufreinigungsverfahren kann eine Abtrennung von Verunreinigungen nicht nur aus einer der Zusammensetzungen (4) bis (11) erfolgen, sondern auch aus mindestens zwei, vorzugsweise mindestens drei und besonders bevorzugt mindestens vier dieser Zusammensetzungen, wobei eine Abtrennung aus der Zusammensetzung (8) am meisten bevorzugt ist, gefolgt von einer Abtrennung aus der Zusammensetzung (10), wiederum gefolgt von einer Abtrennung aus der Zusammensetzung (4), und wiederum gefolgt von einer Abtrennung aus der Zusammensetzung (5) und wiederum gefolgt von einer Abtrennung aus der Zusammensetzung (7).

Wenn es sich bei der Verunreinigung, die im Verfahrensschritt b) von einer oder mehrere der Zusammensetzungen (1) bis (11) abgetrennt wird, um Maleinsäureanhydrid handelt, so ist bei dem erfindungsgemäßen Verfahren sicherzustellen, dass die Zusammensetzungen ausreichende Mengen an Wasser enthalten, um bei einem kontinuierlichen Aufreinigungsverfahren, bei dem abgetrennte Mutterlauge und oder abgetrennte (Meth)Acrylsäurekristalle zumindest teilweise einer der Verfahrensstufen A oder B (oder bei einem Verfahren mit zweistufigem Aufreinigungsverfahren einer der Verfahrensstufen A bis D) zurückgeführt werden, eine ausreichende Hydrolyse der Maleinsäure sicherzustellen. Enthält eine Zusammensetzung für eine zufriedenstellende Abtrennung des Maleinsäureanhydrids nicht genügend Wasser, so kann in dem erfindungsgemäßen Verfahren Wasser zusätzlich eingebracht werden. Dabei kann das Wasser bereits vor dem Verfahrensschritt A dem im Verfahrensschritt A eingesetzten, verunreinigten Roh-(Meth)Acrylsäurestrom aus einem Verfahren zur Herstellung von (Meth)Acrylsäure zugesetzt werden. Die Erhöhung der Wassermenge in dieser Zusammensetzung wirkt sich bei einem kontinuierlich arbeitenden Aufreinigungsverfahren auf den Wassergehalt aller anderen Zusammensetzungen (1) bis (6) und (8) bis (11) aus, so dass das Ausmaß der Kristallisation von Maleinsäure und/oder Fumarsäure in diesen Zusammensetzungen über den Wassergehalt der im Verfahrensschritt A eingesetzten Ausgangszusammensetzung reguliert werden kann. Denkbar ist auch, das Wasser unmittelbar den einzelnen Zusammensetzungen (1) bis (11) zuzusetzen.

Neben der Beeinflussung des Ausmaßes der Hydrolyse des Maleinsäureanhydrids über die Wassermenge in der Zusammensetzung kann dieses auch über Zeit, in der das Maleinsäureanhydrid in einem kontinuierlichen Aufreinigungsverfahren durch die Rückführung von Mutterlauge und/oder (Meth)Acrylsäure im Kreislauf geführt wird, reguliert werden. Diese Dauer ist wiederum abhängig von den relativen Mengen, in denen die Mutterlauge bzw. die gegebenenfalls aufgeschmolzenen (Meth)Acrylsäurekristalle in einzelne Verfahrensstufen zurückgeführt werden. Der Fachmann wird die einzelnen Verfahrensparameter, die zu einer zufriedenstellenden Ausfällung von Maleinsäure und/oder Fumarsäure aus den Zusammensetzungen erforderlich sind, durch einfache Routineversuche ermitteln.

Die vorliegende Erfindung betrifft auch eine Vorrichtung zur Herstellung von Acrylsäure umfassend als fluidleitend miteinander verbundene Komponenten einen Acrylsäure-Reaktor, einen Quenchtower, eine Destillationsvorrichtung und eine Aufreinigungsvorrichtung, die eine Abtrennvorrichtung zum Abtrennen von Verunreinigungen aufweist, welche die Merkmale (ε1) bis (ε5) umfasst:
(s1) die Vorrichtungseinheit weist einen Kristallisationsbereich, vorzugsweise umfassend einen Kristallsuspensionserzeuger, einen Trennbereich, vorzugsweise umfassend eine Waschkolonne, eine Abtrennvorrichtung sowie mindestens zwei Führungen sowie gegebenenfalls mindestens einen Aufschmelzer auf;
(ε2) der Kristallisationsbereich weist mindestens einen Einlass ε2E_1 und einen Auslass ε2A_1 auf, wobei der Auslass ε2A_1 durch eine erste Führung mit einem Einlass ε3E_1 des Trennbereiches ε3 verbunden ist;
(ε3) der Trennbereich weist mindestens Einlass ε3E_1 und mindestens einen Auslass ε3A_1 auf, wobei der Einlass ε3E_1 des Trennbereiches ε3 über die erste Führung mit dem Auslass ε2A_1 des Kristallisationsbereiches verbunden ist und der Auslass ε3A_1 mit einer zweiten Führung für die im Trennbereich abgetrennte Mutterlauge verbunden ist;
(ε4) der Aufschmelzer weist einen Einlass ε4E und einen Auslass ε4A auf, wobei der Einlass ε4E des Aufschmelzers durch eine dritte Führung mit einem weiteren Auslass ε3A_2 des Trennbereiches für die Entnahme abgetrennter (Meth)Acrylsäurekristalle und der Auslass ε4A über eine vierte Führung mit einem weiteren Einlass ε3E_2 des Trennbereiches für die Rückführung aufgeschmolzener (Meth)Acrylsäurekristalle in den Trennbereich verbunden ist oder wobei der Einlass ε4E des Aufschmelzers durch eine fünfte Führung mit einem weiteren Auslass ε3A_2 des Kristallisationsbereiches für die Entnahme einer Kristallsuspension und der Auslass ε4A über eine sechste Führung mit einem weiteren Einlass ε2E_2 des Kristallisationsbereiches verbunden ist;
(ε5) durch die Abtrennvorrichtung wird die zweite Führung, die vierte Führung oder die sechste Führung geleitet, wobei vorzugsweise die zweite, die vierte oder die sechste Führung derart durch die Abtrennvorrichtung geleitet werden, dass eine Abtrennung kristallisierter Verunreinigungen aus den in der zweiten, vierten oder sechsten Führung geführten Zusammensetzungen ermöglicht wird.

Es ist erfindungsgemäß bevorzugt, dass die Abtrennungsvorrichtung (ε5) ein Filter, eine Zentrifuge, eine Sedimentationsvorrichtung oder ein Hydrozyklon ist, wobei ein Filter ganz besonders bevorzugt ist. Als Filter können dabei alle Filter eingesetzt werden, die es ermöglichen, aus einer Zusammensetzung ausgefallene Verunreinigungen diskontinuierlich oder kontinuierlich, vorzugsweise kontinuierlich abzutrennen. In diesem Zusammenhang umfassen besonders bevorzugte, kontinuierlich arbeitende Filter insbesondere Kies- oder Sandfilter, Filternutschen, Kerzenfilter, Blattfilter wie Kreis- oder Axialblattfilter, Zentrifugalscheibenfilter wie etwa Anschwemm- oder Rückstandsfilter, Filterpressen. Bevorzugte kontinuierlich arbeitende Filter umfassen insbesondere Drehfilter, wie etwa Vakuumdrehfilter, Druckdrehfilter oder Trommelfilter, Rotationsdruckfilter, insbesondere solche, die nach dem Prinzip der dynamischen Querstromfiltration arbeiten, Bandfilter wie etwa Vakuumbandfilter, Bandzellenfilter, Kapillarbandfilter oder Papiervlies-Bandfilter.

Die in dem erfindungsgemäßen Verfahren vorzugsweise eingesetzten Filter können sowohl Schüttschichten als auch Flächenfilter umfassen. Als Materialien für die Schüttungen oder die Flächenfilter können alle dem Fachmann bekannten Materialien eingesetzt werden, die eine hinreichende Resistenz gegenüber einem chemischen Angriff vor allem durch die (Meth)Acrylsäure aufweisen.

In diesem Zusammenhang ist es weiterhin bevorzugt, dass es sich bei einem Einsatz eines Flächenfilters um einen Filter, umfassend ein Sieb, vorzugsweise ein Sieb aus Edelstahl, handelt, welches eine Maschenweite in einem Bereich von 0,1 bis 10.000 µm, vorzugsweise in einem Bereich von 10 bis 1.000 µm und besonders bevorzugt in einem Bereich von 100 bis 500 µm aufweist.

In einer bevorzugten Ausführungsform der erfindungsgemäßen Vorrichtung umfasst diese zwei der vorstehend genannten Aufreinigungsvorrichtung, wobei diese beiden Aufreinigungsvorrichtungen derart fluidleitend miteinander verbunden sind, dass die zweite Führung, welche mit dem Auslass ε3A_1 des Trennbereiches der ersten Aufreinigungsvorrichtung verbunden ist, in den Einlass 2E_1 der Kristallisationsvorrichtung der zweiten Aufreinigungsvorrichtung mündet, wobei der Trennbereich der zweiten Aufreinigungsvorrichtung über eine siebte Führung, mit der im Trennbereich der zweiten Aufreinigungsvorrichtung abgetrennte (Meth)Acrylsäurekristalle aus dem Trennbereich der zweiten Abtrennungsvorrichtung abgezogen und in den Kristallisationsbereich der ersten Aufreinigungsvorrichtung geleitet werden können.

Weiterhin betrifft die vorliegende Erfindung die Verwendung der vorstehend beschriebenen Vorrichtung, der vorstehend genannten Aufreinigungsvorrichtung sowie des vorstehend genannten Verfahrens zur Herstellung von (Meth)Acrylsäure, vorzugsweise zur Herstellung von Acrylsäure, die eine Reinheit von mehr als 90 Gew.-%, bevorzugt mehr als 95 Gew.-% und besonders bevorzugt mehr als 99,5 Gew.-%, jeweils bezogen auf die Acrylsäure mit Verunreinigungen, aufweist.

Die Erfindung betrifft auch die nach dem vorstehend genannten Verfahren erhältliche (Meth)Acrylsäure.

Weiterhin betrifft die vorliegende Erfindung (Meth)Acrylsäure, wobei die (Meth)Acrylsäure einen Gehalt an Maleinsäureanhydrid von weniger als 50 ppm, vorzugsweise weniger als 10 ppm und darüber hinaus noch mehr bevorzugt weniger als 1 ppm aufweist.

Auch betrifft die vorliegende Offenbarung wasserabsorbierende Polymere, die durch Polymerisation der vorstehend genannten Acrylsäure erhältlich sind.

Schließlich betrifft die vorliegende Offenbarung auch die Verwendung dieser wasserabsorbierenden Polymere oder der vorstehend genannten (Meth)Acrylsäure, insbesondere der vorstehend genannten Acrylsäure, in Fasern, Folien, Schäumen und Verbunden sowie Fasern, Folien, Schäume und Verbunde, welche die vorstehend genannten wasserabsorbierenden Polymere oder die vorstehend genannte (Meth)Acrylsäure, insbesondere die vorstehend genannte Acrylsäure, beinhalten.
Fig. 1 zeigt die Basis-Lösung des erfindungsgemäßen Verfahrens, bei dem aus einer in einem Behältnis gelagerten Zusammensetzung kontinuierlich kristallisierte Verunreinigungen abgetrennt werden.
Fig. 2A zeigt den Verfahrensablauf eines erfindungsgemäßen Verfahrens, bei dem in einem einstufigen Aufreinigungsverfahren Verunreinigungen aus aufgeschmolzener (Meth)Acrylsäure, die nach Abtrennung aus einer Kristallsuspension erhalten wurden, abgetrennt werden.
Figur 2B zeigt den in Fig. 2A dargestellten Verfahrensablauf, wobei jedoch die Abtrennung der Verunreinigungen nicht direkt innerhalb des Produktkreislaufes, sondern mittels eines separaten, mit dem Produktkreislauf verbundenen Kreislaufes erfolgt.
Fig. 2C zeigt den Verfahrensablauf eines erfindungsgemäßen Verfahrens, bei dem in einem einstufigen Aufreinigungsverfahren Verunreinigungen aus der Kristallsuspension, die im Kristaller erhalten wurde und die mittels eines Produktkreislaufes zumindest teilweise aufgeschmolzen und in den Kristaller zurückgeführt wird, abgetrennt werden.
Fig. 2D zeigt den Verfahrensablauf eines erfindungsgemäßen Verfahrens, bei dem in einem einstufigen Aufreinigungsverfahren Verunreinigungen aus der Mutterlauge, die beim Abtrennen der kristallisierten (Meth)Acrylsäure in der Waschkolonne erhalten wurde und die zumindest teilweise in den Kristallisator zurückgeführt wird, abgetrennt werden.
Fig. 3 zeigt, an welchen Stellen in einem zweistufigen Aufreinigungsverfahren eine erfindungsgemäße Abtrennung von Verunreinigungen erfolgen kann.

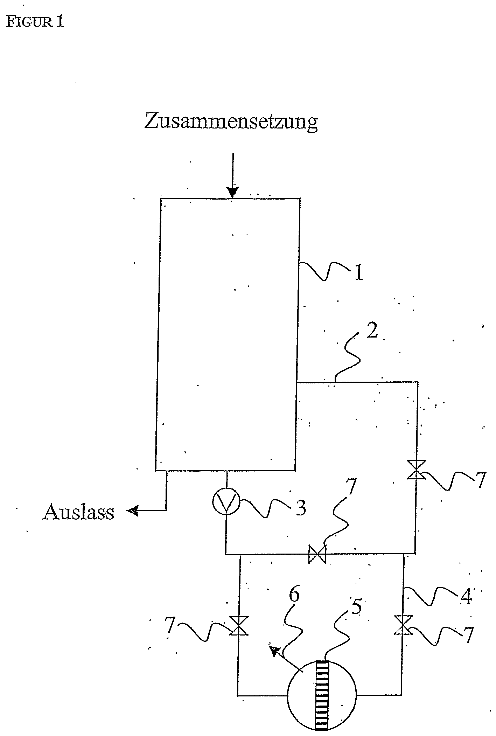

Gemäß der in Fig. 1 dargestellten Basis-Lösung des erfindungsgemäßen Verfahrens wird eine in einem Vorratsbehälter 1 gelagerte Zusammensetzung beinhaltend (Meth)Acrylsäure und Verunreinigungen, insbesondere Maleinsäureanhydrid, so lange gelagert, bis eine ausreichende Hydrolyse des Maleinsäureanhydrids erfolgt ist. Im Falle eines kontinuierlichen Kristallisationsverfahrens zur Aufreinigung der (Meth)Acrylsäure entspricht der Vorratsbehälter 1 einem Suspensionserzeuger, einer Waschkolonne oder einer Zu- oder Ableitung für eine Kristallsuspension, für aus der Kristallsuspension abgetrennte, kristallisierte (Meth)Acrylsäure oder aber für Mutterlauge, je nachdem, aus welcher Zusammensetzung die kristallisierten Verunreinigungen, insbesondere die kristallisierte Maleinsäure und/oder Fumarsäure, abgetrennt wird.

Über eine Produktkreislauf 2 wird die Zusammensetzung kontinuierlich oder diskontinuierlich aus dem Vorratsbehälter 1 entnommen, wobei der Produktkreislauf mittels einer Produktkreislaufpumpe 3 getrieben wird. Aus dem Produktkreislauf 2 wird über einen weiteren Produktkreislauf 4 die Zusammensetzung durch die Abtrennvorrichtung geleitet, in der kristallisierte Verunreinigungen, insbesondere kristallisierte Maleinsäure und/oder Fumarsäure über den Ablauf 8 abgetrennt werden. Anschließend wird die von den kristallisierten Verunreinigungen befreite Zusammensetzung in den Produktkreislauf 2 und über diesen schließlich in den Vorratsbehälter 1 zurückgeführt. Neben der in der Figur 1 dargestellten Vorgehensweise ist es auch möglich, die im Produktkreislauf 2 geführte Zusammensetzung direkt (und nicht mittels des separaten Produktkreislaufes 4) durch die Abtrennvorrichtung 5 zu leiten).

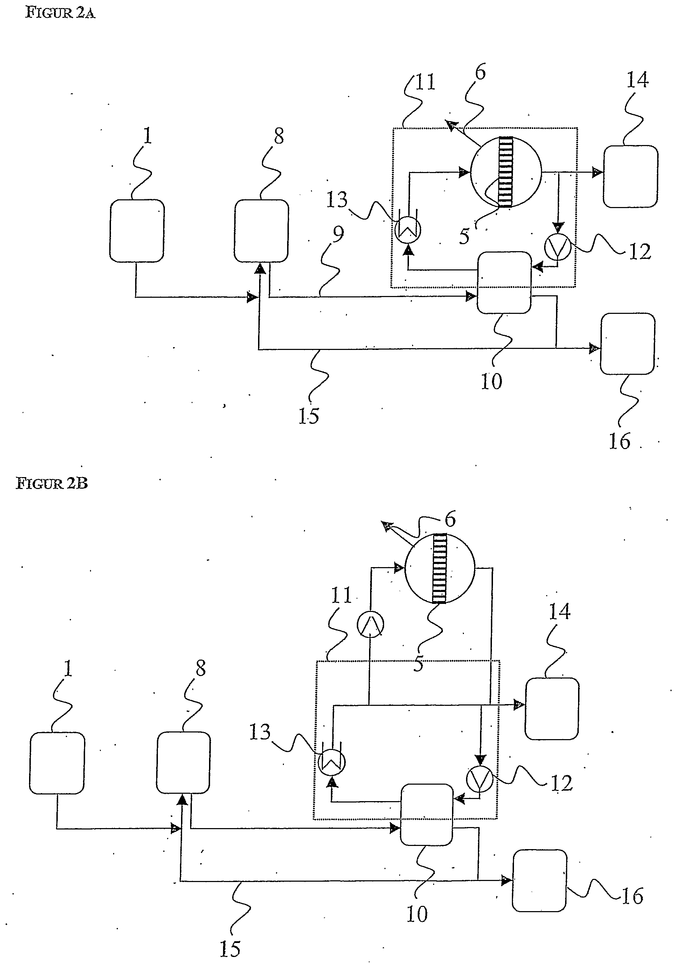

Fig. 2A zeigt den Verfahrensablauf eines erfindungsgemäßen Verfahrens, bei dem in einem einstufigen Aufreinigungsverfahren Verunreinigungen aus der zumindest teilweise aufgeschmolzenen (Meth)Acrylsäure, die nach dem Abtrennen in der Waschkolonne 10 erhalten wurden, abgetrennt werden.

Die im Vorratsbehälter 1 gelagerte Zusammensetzung beinhaltend (Meth)Acrylsäure und Verunreinigungen wird einem Kristallsuspensionserzeuger 8 zugeführt. Die entstehende Kristallsuspension wird anschließend über einen Zulauf 9 in eine Abtrennvorrichtung (z. B. eine Waschkolonne) 10 geleitet. In der Abtrennvorrichtung 10 werden die (Meth)Acrylsäurekristalle von der Mutterlauge getrennt. Außerdem wird ein Teil der (Meth)Acrylsäurekristalle wieder in eine Kristallsuspension überführt und diese Kristallsuspension in einem über eine Produktkreislaufpumpe 12 getriebenen Produktkreislauf 11 geführt, in dem die (Meth)Acrylsäurekristalle mittels eines Wärmeaustauschers 13 aufgeschmolzen und zur Steigerung der Reinheit der (Meth)Acrylsäure zumindest teilweise als Waschflüssigkeit zur Gegenstromwäsche in die Waschkolonne 10 zurückgeführt werden. Somit dient die Waschkolonne 10 zur Fest-Flüssig-Abtrennung sowie zur Durchführung einer Verdrängungswäsche, wobei die Verdrängungswäsche ohne Verlust an Waschflüssigkeit durchgeführt wird. Der andere Teil der (Meth)Acrylsäurekristalle verlässt die Anlage und strömt in den Produktbehälter 14 (genügt die Reinheit der mittels des in der Abbildung 2A dargestellten einstufigen Aufreinigungsverfahrens erhaltenen (Meth)Acrylsäure nicht, so können die (Meth)Acrylsäurekristalle einer weiteren Reinigungsstufe zugeführt werden). Die zumindest teilweise aufgeschmolzenen (Meth)Acrylsäurekristalle werden durch die Abtrennvorrichtung zur Abtrennung von Verunreinigungen 5 geführt. Gemäß der Figur 2B können die zumindest teilweise aufgeschmolzenen (Meth)Acrylsäurekristalle auch mittels eines separaten Produktkreislaufes durch die Abtrennvorrichtung geleitet werden. Die abgetrennten Verunreinigungen werden über die Ableitung 6 entfernt und die von den Verunreinigungen befreite Zusammensetzung wird in die Abtrennvorrichtung 10 zurückgeführt. In einer bevorzugten Ausführungsform dieses einstufigen Aufreinigungsverfahrens wird zumindest ein Teil der in der Waschkolonne 10 abgetrennten Mutterlauge über den Zulauf 15 in den Suspensionserzeuger 8 zurückgeführt, der andere Teil der Mutterlauge wird als Abstoß in den Mutterlaugenbehälter 16 geleitet.

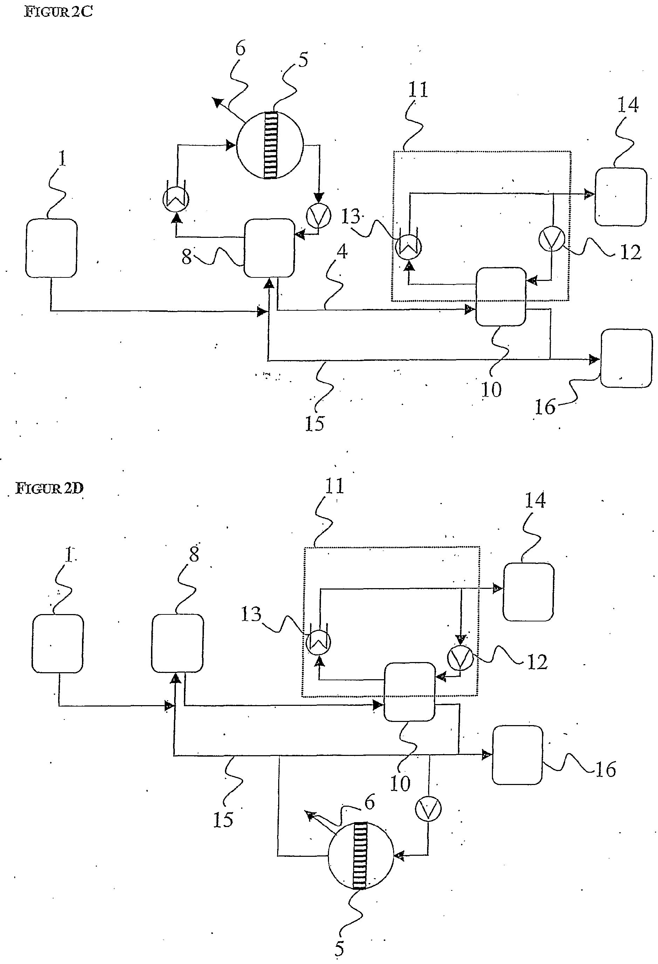

Die Figuren 2C und 2D zeigen das der Figur 2A entsprechende einstufige Aufreinigungsverfahren, wobei jedoch nicht die zumindest teilweise aufgeschmolzenen (Meth)Acrylsäurekristalle, die nach dem Abtrennen in der Waschkolonne 10 erhalten wurden, sondern die im Suspensionserzeuger 8 erhaltene Kristallsuspension (Figur 2D) oder die in den Suspensionserzeuger 8 zurückgeführte Mutterlauge der Abtrennvorrichtung 5 zugeführt werden.

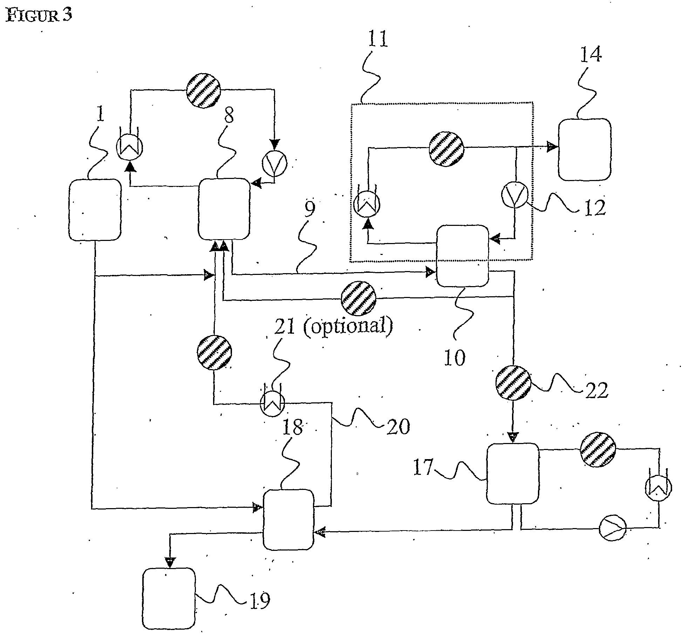

Figur 3 zeigt ein zweistufiges Aufreinigungsverfahren, bei dem die in der Waschkolonne 8 abgetrennte Mutterlauge zumindest teilweise in einen weiteren Suspensionserzeuger 17 geleitet wird. Die im Suspensionserzeuger 17 erhaltene Kristallsuspension wird in eine weitere Waschkolonne 18 geführt, in der die (Meth)Acrylsäurekristalle von der Mutterlauge getrennt werden. Die in dieser zweiten Waschkolonne 18 abgetrennten (Meth)Acrylsäurekristalle werden in kristalliner Form oder nachdem sie zumindest teilweise mittels eines Wärmeaustauschers 18 aufgeschmolzen wurden, über die Zuleitung 20 dem ersten Suspensionserzeuger zugeführt. Es ist in diesem zweistufigen Aufreinigungsverfahren auch bevorzugt, dass die Roh-(Meth)Acrylsäure aus dem Vorratsbehälter 1 in den Kopf der zweiten Waschkolonne geleitet wird, um auf diese Weise die in der zweiten Verfahrensstufe abgeschabten Produktkristalle als Kristallsuspension in den Suspensionserzeuger der Verfahrensstufe A zu führen. Diese Variante hat den energetischen Vorteil, auf ein Aufschmelzen in der zweiten Stufe verzichten zu können und die nun in der ersten Stufe vorhandenen Kristalle nicht noch einmal ausfrieren zu müssen.

Veranschaulicht ist in der Figur 3, an welchen Stellen eine Abtrennung von kristallisierten Verunreinigungen, insbesondere von kristallisierter Maleinsäure und/oder Fumarsäure, mittels der Abtrennvorrichtung 22 möglich ist.

Die Erfindung wird nun anhand eines Beispiels näher erläutert:

### BEISPIEL

113,5 g eines Sumpfproduktes aus einer Kolonne mit der in der Tabelle 1 angegebenen Zusammensetzung wurde mit 1,6 g Wasser in einem Erlenmeyerkolben vorgelegt. Die Mischung wurde über einen Zeitraum von 42 Stunden bei Raumtemperatur stehengelassen und anschließend über eine Vakuumnutsche filtriert. 108,5 g Filtrat mit der ebenfalls in der Tabelle 1 angegebenen Zusammensetzung wurden erhalten. Der auf dem Filter verbleibende Feststoff wurde anschließend bei 4.000 upm für 5 Minuten zentrifugiert, wobei 3,6 g eines Feststoffes erhalten wurden. Dieser Feststoff wurde zum Zwecke der Analyse in 129,0 g hochreiner Acrylsäure gelöst. Die Zusammensetzung des Feststoffes ist ebenfalls in der Tabelle 1 angegeben (die in den Zusammensetzungen enthaltenen Komponenten wurden jeweils mittels Gaschromatographie und Karl-Fischer-Titration analysiert; die rel. Mengen der Komponenten im Sumpfprodukt, im Filtrat und im Feststoff summieren sich, bedingt durch das Auf- und Abrunden der für diese Zusammensetzungen erhaltenen Analysedaten nicht genau auf 100 Gew.-%).

**Tabelle 1**

| Komponente (alle Angaben, sofern nicht anders angegeben, in Gew.-%) | Sumpfprodukt (bezogen auf die Gesamtmenge des Sumpfproduktes) | Filtrat (bezogen auf die Gesamtmenge des Filtrates) | Feststoff (bezogen auf die Gesamtmenge des Feststoffes) |
|---|---|---|---|
| Wasser | 0,018 | 0,979 | 1,22 |
| MEHQ¹⁾ | 0,0044 | 0,0037 | 0,51 |
| Hydrochinon | 0,113 | 0,125 | < 800 ppm |
| Acrylsäure | 88,6 | 89,0 | 48,43 |
| Essigsäure | 0,055 | 0,049 | 0,59 |
| Propionsäure | 0,036 | 0,034 | 0,48 |
| Dimere Acrylsäure | 2,759 | 2,959 | 4,79 |
| MA/MS²⁾ | 8,131 | 6,215 | 44,16 |
| Furfural | 0,0327 | 0,036 | < 40 ppm |
| Benzaldehyd | 0,075 | 0,08 | < 40 ppm |
| Acrolein | 0,001 | 0,0019 | < 40 ppm |
| Protoanemonin | 0,039 | 0,0398 | < 40 ppm |
| Rest | 0,06 | 0,440 | < 800 ppm |

| | | | |
|---|---|---|---|
| ¹⁾ Methylhydrochinon ²⁾ Maleinsäureanhydrid/Maleinsäure | | | |

## Patentansprüche

1. Ein Verfahren zur Herstellung von (Meth)Acrylsäure, wobei zunächst eine Roh-(Meth)Acrylsäure hergestellt und diese Roh-(Meth)Acrylsäure anschliessend kontinuierlich aufgereinigt wird, wobei die kontinuierliche Aufreinigung der Roh-(Meth)Acrylsäure folgende Verfahrensschritte beinhaltet:
a) in einer Zusammensetzung beinhaltend (Meth)Acrylsäure und Verunreinigungen, wobei es sich bei den Verunreinigungen um eine Mischung von Maleinsäure, Fumarsäure und Maleinsäureanhydrid handelt, werden die Verunreinigungen in kristalliner Form aus der Zusammensetzung ausgefällt;
b) die aus der Zusammensetzung ausgefallenen kristallinen Verunreinigungen werden abgetrennt,
wobei das Ausfällen des Maleinsäureanhydrids im Schritt a) durch kontrollierte Hydrolyse des Maleinsäureanhydrids über eine Zeitraum von 1 bis 250 Stunden erfolgt.

2. Verfahren nach Anspruch 1, wobei die Abtrennung des Maleinsäureanhydrids aus der Zusammensetzung dadurch erfolgt, dass im Verfahrensschritt a) Maleinsäureanhydrid zu einem Anteil von mindestens 50 Mol-%, bezogen auf die Gesamtmenge des vor Beginn der Hydrolyse in der Zusammensetzung enthaltenen Maleinsäureanhydrids, unter Bildung von Maleinsäure und/oder Fumarsäure hydrolysiert wird, wobei Maleinsäure und/oder Fumarsäure in kristalliner Form aus der Zusammensetzung ausfallen.

3. Verfahren nach Anspruch 1, wobei die Hydrolyse des Maleinsäureanhydrids im Verfahrensschritt a) über einen Zeitraum in einem Bereich von 1 bis 250 Stunden bei einer Temperatur in einem Bereich von -70 bis 130°C erfolgt.

4. Verfahren nach einem der vorhergehenden Ansprüche, wobei es sich bei der Zusammensetzung um die (Meth)Acrylsäurekristalle in vorzugsweise mindestens teilweise aufgeschmolzener Form oder um die Mutterlauge handelt, die im Schritt B oder im Schritt D eines Verfahrens erhalten werden, welches folgende Verfahrensschritte beinhaltet:
A) Kristallisation von (Meth)Acrylsäure aus einem verunreinigten Roh-(Meth)Acrylsäurestrom aus einem Verfahren zur Herstellung von (Meth)Acrylsäure unter Bildung einer Kristallsuspension mittels eines ersten Suspensionserzeugers;
B) Abtrennen der (Meth)Acrylsäurekristalle aus der Kristallsuspension mittels einer ersten Abtrennvorrichtung, wobei eine Mutterlauge zurückbehalten wird; sowie gegebenenfalls
C) erneute Kristallisation von (Meth)Acrylsäure aus der im Schritt B) erhaltenen ersten Mutterlauge unter Bildung einer zweiten Kristallsuspension mittels eines zweiten Suspensionserzeugers;
D) Abtrennen der im Schritt C) erhaltenen (Meth)Acrylsäurekristalle aus der zweiten Kristallsuspension mittels einer weiteren Abtrennvorrichtung, wobei eine zweite Mutterlauge zurückbehalten wird.

5. Verfahren nach einem der vorhergehenden Ansprüche, wobei es sich bei der Zusammensetzung um die Kristallsuspension in vorzugsweise mindestens teilweise aufgeschmolzener Form handelt, die im Verfahrensschritt A oder Schritt C eines Verfahrens erhalten wird, welches folgende Verfahrensschritte beinhaltet:
A) Kristallisation von (Meth)Acrylsäure aus einem verunreinigten Roh-(Meth)Acrylsäurestrom aus einem Verfahren zur Herstellung von (Meth)Acrylsäure unter Bildung einer Kristallsuspension mittels eines ersten Suspensionserzeugers;
B) Abtrennen der (Meth)Acrylsäurekristalle aus der Kristallsuspension mittels einer ersten Abtrennvorrichtung, wobei eine Mutterlauge zurückbehalten wird; sowie gegebenenfalls
C) erneute Kristallisation von (Meth)Acrylsäure aus der im Schritt B) erhaltenen ersten Mutterlauge unter Bildung einer zweiten Kristallsuspension mittels eines zweiten Suspensionserzeugers;
D) Abtrennen der im Schritt C) erhaltenen (Meth)Acrylsäurekristalle aus der zweiten Kristallsuspension mittels einer weiteren Abtrennvorrichtung, wobei eine zweite Mutterlauge zurückbehalten wird.

6. Eine Vorrichtung zur Herstellung von Acrylsäure umfassend als fluidleitend miteinander verbundene Komponenten einen Acrylsäure-Reaktor, einen Quenchtower, eine Destillationsvorrichtung und eine Aufreinigungsvorrichtung, die eine Abtrennvorrichtung zum Abtrennen von Verunreinigungen aufweist, welche die Merkmale (ε1) bis (ε5) umfasst:
(ε1) die Vorrichtungseinheit weist einen Kristallisationsbereich, einen Trennbereich, eine Abtrennvorrichtung, mindestens zwei Führungen sowie einen Aufschmelzer, auf;
(ε2) der Kristallisationsbereich weist mindestens einen Einlass ε2E_1 für einen verunreinigten (Meth)Acrylsäurestrom und einen Auslass ε2A_1 für eine Kristallsuspension auf, wobei der Auslass ε2A_1 durch eine erste Führung mit einem Einlass ε3E_1 des Trennbereiches ε3 verbunden;
(ε3) der Trennbereich weist mindestens einen Einlass ε3E_1 und mindestens einen Auslass ε3A_1 auf, wobei der Einlass ε3E_1 des Trennbereiches ε3 über die erste Führung mit dem Auslass ε2A_1 des Kristallisationsbereiches verbunden ist und der Auslass ε3A_1 mit einer zweiten Führung für die im Trennbereich abgetrennte Mutterlauge verbunden ist;
(ε4) der Aufschmelzer weist einen Einlass ε4E und einen Auslass ε4A auf, wobei der Einlass ε4E des Aufschmelzers durch eine dritte Führung mit einem weiteren Auslass ε3A_2 des Trennbereiches für die Entnahme abgetrennter (Meth)Acrylsäurekristalle und der Auslass ε4A über eine vierte Führung mit einem weiteren Einlass ε3E_2 des Trennbereiches verbunden ist oder wobei der Einlass ε4E des Aufschmelzers durch eine fünfte Führung mit einem weiteren Auslass ε3A_2 des Kristallisationsbereiches für die Entnahme einer Kristallsuspension und der Auslass ε4A über eine sechste Führung mit einem weiteren Einlass ε2E_2 des Kristallisationsbereiches verbunden ist;
(ε5) durch die Abtrennvorrichtung wird die zweite Führung, die vierte Führung oder die sechste Führung geleitet.

7. Vorrichtung nach Anspruch 6, wobei die Abtrennungsvorrichtung (ε5) ein Filter ist.

## Claims

1. A process for preparing (meth)acrylic acid, wherein a crude (meth) acrylic acid is first prepared and this crude (meth)acrylic acid is then continuously purified, wherein the continuous purification of the crude (meth)acrylic acid comprises the following process steps:
a) in a composition comprising (meth) acrylic acid and impurities, the impurities being a mixture of maleic acid, fumaric acid and maleic anhydride, precipitating the impurities out of the composition in crystalline form;
b) separating the crystalline impurities precipitated out of the composition,
wherein the precipitating of the maleic anhydride in step a) is effected by controlled hydrolysis of the maleic anhydride over a period of 1 to 250 hours.

2. Process according to Claim 1, wherein the maleic anhydride is separated out of the composition by, in process step a), hydrolysing the maleic anhydride in a proportion of at least 50 mol%, based on the total amount of the maleic anhydride present in the composition prior to commencement of the hydrolysis, forming maleic acid and/or fumaric acid, wherein maleic acid and/or fumaric acid precipitate out of the composition in crystalline form.

3. Process according to Claim 1, wherein the maleic anhydride is hydrolysed in process step a) over a period of time within a range from 1 to 250 hours at a temperature within a range from -70 to 130°C.

4. Process according to any of the preceding claims, wherein the composition is the (meth)acrylic acid crystals in preferably at least partly molten form or the mother liquor which are obtained in step B or in step D of a process comprising the following process steps:
A) crystallizing (meth)acrylic acid out of a contaminated crude (meth)acrylic acid stream from a process for preparing (meth)acrylic acid to form a crystal suspension by means of a first suspension generator;
B) separating the (meth)acrylic acid crystals from the crystal suspension by means of a first separation apparatus, with retention of a mother liquor; and optionally
C) again crystallizing (meth)acrylic acid out of the first mother liquor obtained in step B) to form a second crystal suspension by means of a second suspension generator;
D) separating the (meth)acrylic acid crystals obtained in step C) out of the second crystal suspension by means of a further separation apparatus, with retention of a second mother liquor.

5. Process according to any of the preceding claims, wherein the composition is the crystal suspension in preferably at least partly molten form which is obtained in process step A or step C of a process comprising the following process steps:
A) crystallizing (meth)acrylic acid out of a contaminated crude (meth)acrylic acid stream from a process for preparing (meth)acrylic acid to form a crystal suspension by means of a first suspension generator;
B) separating the (meth)acrylic acid crystals from the crystal suspension by means of a first separation apparatus, with retention of a mother liquor; and optionally
C) again crystallizing (meth)acrylic acid out of the first mother liquor obtained in step B) to form a second crystal suspension by means of a second suspension generator;
D) separating the (meth)acrylic acid crystals obtained in step C) out of the second crystal suspension by means of a further separation apparatus, with retention of a second mother liquor.

6. An apparatus for preparation of acrylic acid comprising, as components connected with each other in a fluid-conducting manner, an acrylic acid reactor, a quench tower, a distillation apparatus and a purification apparatus having a separation apparatus for separation of impurities, which comprises the features (ε1) to (ε5):
(ε1) the apparatus unit comprises a crystallization region, a separation region, a separation apparatus, at least two feeds and a melter;
(ε2) the crystallization region comprises at least one inlet ε2E_1 for a contaminated (meth)acrylic acid stream and an outlet ε2A_1 for a crystal suspension, wherein the outlet ε2A_1 is connected by a first feed to an inlet ε3E_1 of the separation region ε3;
(ε3) the separation region has at least one inlet ε3E_1 and at least one outlet ε3A_1, the inlet ε3E_1 of the separation region ε3 being connected via the first feed to the outlet ε2A_1 of the crystallization region and the outlet ε3A_1 being connected to a second feed for the mother liquor separated off in the separation region;
(ε4) the melter has an inlet ε4E and an outlet ε4A, the inlet ε4E of the melter being connected by a third feed to a further outlet ε3A_2 of the separation region for the removal of separated (meth)acrylic acid crystals and the outlet ε4A being connected via a fourth feed to a further inlet ε3E_2 of the separation region, or the inlet ε4E of the melter being connected by a fifth feed to a further outlet ε3A_2 of the crystallization region for the removal of a crystal suspension and the outlet ε4A being connected via a sixth feed to a further inlet ε2E_2 of the crystallization region;
(ε5) the second feed, the fourth feed or the sixth feed is routed through the separation apparatus.

7. Apparatus according to Claim 6, wherein the separation apparatus (ε5) is a filter.

## Revendications

1. Procédé pour la production d'acide (méth)acrylique, dans lequel on produit d'abord un acide (méth)acrylique brut et cet acide (méth)acrylique brut est ensuite purifié en continu, la purification continue de l'acide (méth)acrylique brut comprenant les étapes de processus suivantes :
a) dans une composition comprenant de l'acide (méth)acrylique et des impuretés, les impuretés consistant en un mélange d'acide maléique, d'acide fumarique et d'anhydride maléique, on fait précipiter sous forme cristalline les impuretés de la composition ;
b) on sépare de la composition les impuretés cristallines précipitées,
la précipitation de l'anhydride maléique dans l'étape a) s'effectuant par hydrolyse contrôlée de l'anhydride maléique pendant une durée de 1 à 250 heures.

2. Procédé selon la revendication 1, dans lequel la séparation de l'anhydride maléique d'avec la composition a lieu par le fait que dans l'étape a) du processus l'anhydride maléique est hydrolyse à un degré d'au moins 50 % en moles, par rapport à la quantité totale de l'anhydride maléique contenu dans la composition avant le début de l'hydrolyse, avec formation d'acide maléique et/ou d'acide fumarique, l'acide maléique et/ou l'acide fumarique se séparant de la composition en précipitant sous forme cristalline.

3. Procédé selon la revendication 1, dans lequel l'hydrolyse de l'anhydride maléique dans l'étape a) du processus s'effectue pendant une durée dans une plage de 1 à 250 heures à une température dans une plage de -70 à 130 °C.

4. Procédé selon l'une quelconque des revendications précédentes, dans lequel pour ce qui est de la composition il s'agit des cristaux d'acide (méth)acrylique de préférence sous forme au moins partiellement fondue ou de la liqueur mère, qui sont obtenus dans l'étape B ou dans l'étape D d'un procédé qui comprend les étapes de processus suivantes :
A) cristallisation d'acide (méth)acrylique à partir d'un courant d'acide (méth)acrylique impur provenant d'un procédé destiné à la production d'acide (méth)acrylique, avec formation d'une suspension de cristaux au moyen d'un premier générateur de suspension ;
B) séparation des cristaux d'acide (méth)acrylique d'avec la suspension de cristaux au moyen d'un premier dispositif de séparation ; de sorte qu'une liqueur mère est retenue ; ainsi qu'éventuellement
C) nouvelle cristallisation d'acide (méth)acrylique à partir de la première liqueur mère obtenue dans l'étape B), avec formation d'une deuxième suspension de cristaux au moyen d'un deuxième générateur de suspension ;
D) séparation des cristaux d'acide (méth)acrylique obtenus dans l'étape C) d'avec la deuxième suspension de cristaux au moyen d'un autre dispositif de séparation, de sorte qu'une deuxième liqueur mère est retenue.

5. Procédé selon l'une quelconque des revendications précédentes, dans lequel pour ce qui est de la composition il s'agit de la suspension de cristaux de préférence sous forme au moins partiellement fondue, qui est obtenue dans l'étape A du procédé ou l'étape C d'un procédé qui comprend les étapes de processus suivantes :
A) cristallisation d'acide (méth)acrylique à partir d'un courant d'acide (méth) acrylique impur provenant d'un procédé destiné à la production d'acide (méth)acrylique, avec formation d'une suspension de cristaux au moyen d'un premier générateur de suspension ;
B) séparation des cristaux d'acide (méth)acrylique d'avec la suspension de cristaux au moyen d'un premier dispositif de séparation, de sorte qu'une liqueur mère est retenue ; ainsi qu'éventuellement
C) nouvelle cristallisation d'acide (méth)acrylique à partir de la première liqueur mère obtenue dans l'étape B), avec formation d'une deuxième suspension de cristaux au moyen d'un deuxième générateur de suspension ;
D) séparation des cristaux d'acide (méth)acrylique obtenus dans l'étape C) d'avec la deuxième suspension de cristaux au moyen d'un autre dispositif de séparation, de sorte qu'une deuxième liqueur mère est retenue.

6. Dispositif pour la production d'acide acrylique, comprenant en tant que composants en communication fluidique entre eux un réacteur d'acide acrylique, une tour de refroidissement, un dispositif de distillation et un dispositif de purification qui comprend un dispositif de séparation destiné à la séparation d'impuretés, qui comprend les caractéristiques (ε1) à (ε5) suivantes :
(ε1) l'unité d'appareillage comporte une zone de cristallisation, une zone de séparation, un dispositif de séparation, au moins deux conduits, ainsi qu'un dispositif de fusion ;
(ε2) le zone de cristallisation comporte au moins une entrée ε2E_1 pour un courant d'acide (méth)acrylique impur et une sortie ε2A_1 pour une suspension de cristaux, la sortie ε2A_1 étant reliée à une entrée ε3E_1 de la zone de séparation ε3 par un premier conduit ;
(ε3) la zone de séparation comporte au moins une entrée ε3E_1 et au moins une sortie ε3A_1, l'entrée ε3E_1 de la zone de séparation ε3 étant reliée à la sortie ε2A_1 de la zone de cristallisation par le premier conduit et la sortie ε3A_1 étant reliée à un deuxième conduit pour la liqueur mère séparée dans la zone de séparation ;
(ε4) le dispositif de fusion comporte une entrée ε4E et une sortie ε4A, l'entrée ε4E du dispositif de fusion étant reliée par un troisième conduit à une autre sortie ε3A_2 de la zone de séparation pour la décharge de cristaux d'acide (méth)acrylique séparés et la sortie ε4A étant reliée par un quatrième conduit à une autre entrée ε3E_2 de la zone de séparation ou l'entrée ε4E du dispositif de fusion étant reliée par un cinquième conduit à une autre sortie ε3A_2 de la zone de cristallisation pour la décharge d'une suspension de cristaux et la sortie ε4A étant reliée par un sixième conduit à une autre entrée ε2E_2 de la zone de cristallisation ;
(ε5) on fait passer le deuxième conduit, le quatrième conduit ou le sixième à travers le dispositif de séparation.

7. Dispositif selon la revendication 6, dans lequel le dispositif de séparation (ε5) est un filtre.
